# EUROPEAN PATENT APPLICATION

(11) **EP 2 620 159 A1**
(43) Date of publication of application: **31.07.2013**
(21) Application number: 12305086.6
(22) Date of filing: 24.01.2012
(51) Int. Cl.: A61K 39/07

(54) **Improved cancer treatment by immunotherapy with bcg or antigenically related non-pathogenic mycobacteria**

(71) Applicant: Institut Pasteur, 75015 Paris (FR)
(72) Inventor: Biot, Claire, 92160 Antony (FR); Albert, Matthew, 75014 Paris (FR); Rentsch, Cyril, 4125 Riehen (CH); Gsponer, Joël, 4058 Basel (CH)
(74) Representative: Leblois-Préhaud, Hélène Marthe Georgette

(57) **Abstract**

A first and a second identical or different mycobacterial immunogenic compositions, each comprising at least a *Mycobacterium bovis* bacillus Calmette-Guérin (BCG), an antigenically related non-pathogenic mycobacteria, or one or more immunogenic component(s) thereof, as therapeutic active ingredient(s) for use in the treatment of cancer by parenteral or oral administration of the first composition to a cancer patient before local administration of the second composition at tumor site. A method *in vitro* for monitoring cancer treatment by immunotherapy with BCG, antigenically related non-pathogenic mycobacteria, or immunogenic component(s) thereof, comprising assaying BCG-specific immune response in a patient.

SEQUENCE LISTING

<110>  INSTITUT PASTEUR

<120>  Improved cancer treatment by immunotherapy with BCG or
       antigenically related non-pathogenic mycobacteria

<130>  F0226CAS192/EP

<160>  2

<170>  PatentIn version 3.5

<210>  1
<211>  10
<212>  PRT
<213>  artificial sequence

<220>
<223>  Mtb32 309-318 peptide

<400>  1

Gly Ala Pro Ile Asn Ser Ala Thr Ala Met
1               5                   10


<210>  2
<211>  20
<212>  PRT
<213>  artificial sequence

<220>
<223>  Ag85A  241-260 peptide

<400>  2

Gln Asp Ala Tyr Asn Ala Gly Gly Gly His Asn Gly Val Phe Asp Phe
1               5                   10                  15


Pro Asp Ser Gly
            20

## Description

The invention relates to an improved cancer treatment by immunotherapy with BCG, antigenically related non-pathogenic mycobacteria, or immunogenic component(s) thereof, as well as to a method for monitoring cancer treatment by immunotherapy with BCG, antigenically related non-pathogenic mycobacteria, or immunogenic component(s) thereof.

The use of infectious agents for achieving an anti-tumor effect dates back to the 1700s, but it was William Coley who made the first attempt at extracting an active agent for the purpose of achieving immune-mediated regression of inoperable sarcomas. His extract, called Coley's toxin, was a streptococcal extract that was later supplemented with mycobacterial cell wall preparations. Remarkably, he reported ∼10% response rates in patients with advanced stage disease (Wiemann, B. and Starnes, C.O., Pharmacol. Ther., 1994, 64, 529-564).

Bacillus Calmette Guérin (BCG) is a live attenuated strain of *Mycobacterium bovis* generated by the repetitive passage of a virulent strain of *M*. *bovis*. BCG was initially developed as a vaccine for tuberculosis (*Mycobacterium tuberculosis (M. tuberculosis or Mtb)* infection; Zwerling et al., PLoS Med., 2011, 8, e1001012) and following the work of William Coley, BCG was evaluated for use as an anti-cancer therapeutic vaccine. In fact, it has been injected into many solid tumors and while there were reports of some success, controlled clinical trials did not provide statistical significance (Brandau, H. Suttmann, Biomed. Pharmacother., 2007, 61, 299; Mathe et al., Lancet, 1969, 1, 697- ; Morton et al., Ann. Surg., 1970, 172, 740-). Nonetheless, animal studies with BCG continued and it was recognized that long lasting direct contact with the live bacteria resulted in optimal tumor immunity ( Zbar et al., J. Natl. Cancer Inst., 1971, 46, 831-). These results prompted Morales et al. to evaluate BCG as an adjuvant intravesical treatment for carcinoma of the bladder, (Morales et al., J. Urol., 1976, 116, 180-; Herr et al., J. Urol., 2008, 179, 53-).

Carcinoma of the bladder is the most common cancer of the urinary tract and the fourth most common malignant disease in the developed world (Jemal, A. et al, CA Cancer J. Clin., 2011, 61, 69-90). Most tumors are diagnosed at a superficial stage and are surgically removed by transurethral resection (Babjuk, M. et al., Eur. Urol., 2011, 59, 997-). Depending on the stage and grade of the non-muscle invasive tumors, adjuvant therapy is recommended as a strategy for both reducing recurrence and diminishing risk of progression.

The initial treatment schedule was established empirically by Morales and colleagues in 1976 (Morales et al., J. Urol., 1976, 116, 180-): 120mg lyophilized BCG Pasteur was reconstituted in 50mL saline and instilled via a catheter into the bladder. Patients were asked to retain the solution for at least 2 hours, and they additionally received 5mg BCG intradermally. Treatments were given weekly over 6 weeks, and altered favorably the pattern of recurrence in 9 patients.

Since then, BCG therapy has been the standard of care for high-risk urothelial carcinoma, namely carcinoma *in situ,* and high-grade Ta/T1 bladder lesions (Babjuk et al., Eur. Urol., 2011, 59, 997-). It is also the most successful immunotherapy applied in the clinics, with response rates ranging 50-70% in patients with non-muscle invasive bladder cancer.

Modifications of the initial regimen have mainly focused on the elimination of the concomitant intradermal dose, introduction of maintenance BCG dosage schedule and introduction of other substrains of BCG. Importantly, two clinical studies performed in the 1990s have investigated the combined use of intravesical and intradermal (or scarification) routes for treating patients with BCG (Herr H.W., J Urol, 1986, 135, 265-267; Lamm et al., J. Urol., 1991, 145, 738-). These trials showed no evidence of enhanced clinical response so that nowadays the regimen recommended by the European and American guidelines is as follows (Babjuk et al., Eur. Urol., 2011, 59, 997- ; Gontero et al., Eur. Urol., 2010, 57, 410-) :
- BCG therapy should be initiated 2 weeks after transurethral resection of the tumor,
- Any of the commercially available BCG strains (e.g. Connaught, Tice, RIVM) can be proposed for intravesical use,
- The routine procedure is to measure BCG dose in milligrams rather than in number of colony forming units (CFUs, i.e. live bacteria). Depending on the commercial preparation, dose range from 50mg (Tice) to 120mg (Pasteur, not commercially available any more) and are in the range of 10⁸-10⁹ CFUs.
- BCG dwell time in the bladder is 2 hours.
- The induction course of 6 weekly intravesical instillations is followed by maintenance therapy. The recommended maintenance regimen consists in 3 weekly instillations at 3 months, 6 months, and then every 6 months up to 3 year. Of note, the efficacy of such regimen is nowadays debated (Gontero et al., Eur. Urol., 2010, 57, 410-; Herr et al., Eur. Urol., 2011, 60, 32-).

While now in use for over 35 years, many questions remain about the mechanism of action by which BCG mediates the observed clinical response (Brandau et al., Biomed. Pharmacother., 2007, 61, 299-), additionally, there is a need to identify strategies for optimizing therapy.

While success of therapy is known to rely on repeated instillations of live BCG administered as adjuvant therapy shortly after tumor resection, its precise mechanisms of action remain unclear. In the context of bladder cancer, repeated instillation with clinical-grade BCG is known to trigger a strong innate immune response, followed by the influx of type 1 polarized lymphocyte subsets (Alexandroff et al., Immunotherapy, 2010, 2, 551-; Brandau et al., Biomed. Pharmacother. 2007, 61, 299-). Using orthotopically-transplanted urothelial tumors in mice, several groups have reported that BCG-mediated anti-tumor activity relies on a functional immune system of the tumor-bearing host (Ratliff et al., J. Urol., 1987, 137, 155-; Ratliff et al., J. Urol., 1993, 150, 1018- ; Brandau et al., Int. J. Cancer, 2001, 92, 697- ; Suttmann et al., Cancer Res. 2006, 66, 8250-). In particular, CD4⁺ and CD8⁺ T lymphocytes seem to be essential effector cells for eliminating the tumor in a mouse model (Ratliff et al., J. Urol., 1993, 150, 1018-), and correlates have been established between T cell infiltration and clinical response in patients (Prescott et al., J. Urol. 1992, 147, 1636-). The inventors have recently reported that repeated intravesical instillations with BCG were required in order to trigger a robust inflammatory response (Bisiaux et al., J. Urol., 2009, 181, 1571-).

The purified protein derivative (PPD) skin test is a standard assay that is used to detect an active immune response to BCG in subjects previously vaccinated with BCG. Tuberculin skin testing (TST) has been used for years as an aid in diagnosing latent tuberculosis infection (LTBI) and includes measurement of the delayed type hypersensitivity (DTH) response 48-72 hours after intradermal injection of PPD. While not typically attributed to inflammation in the bladder mucosa, DTH reactions are known to be mediated by antigen-specific effector T cells (e.g., induration induced by PPD challenge in the skin of a primed individual). This reaction is defined by antigen-specific T cells mediating the rapid recruitment of inflammatory cells to the site of injection (Marchal et al., J. Immunol., 1982, 129, 954- ; Milon et al., J. Immunol., 1983, 130, 1103-). The PPD skin test has been assessed as potential predictor of BCG response. However, current evidence does no support its use as predictor of BCG response in clinical practice (Gontero et al., Eur. Urol., 2010, 57, 410-).

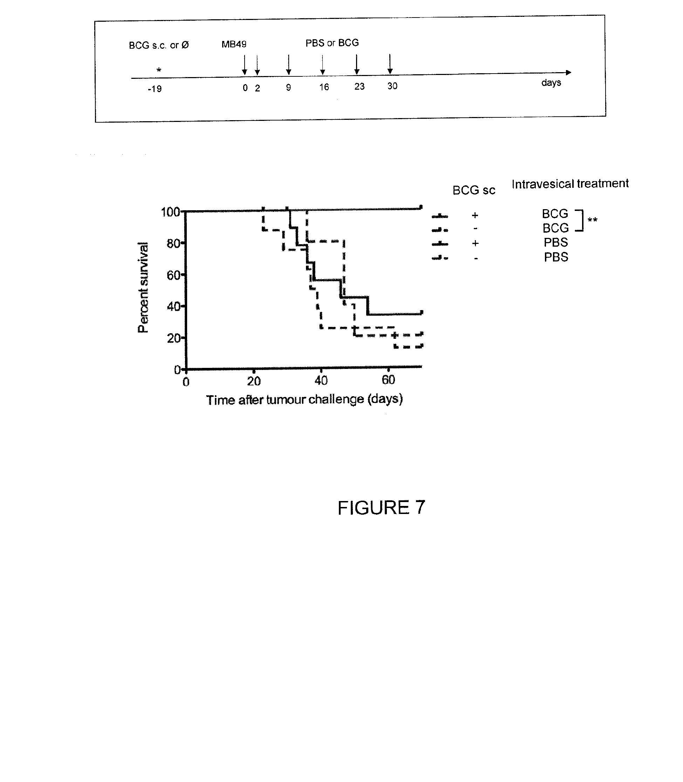

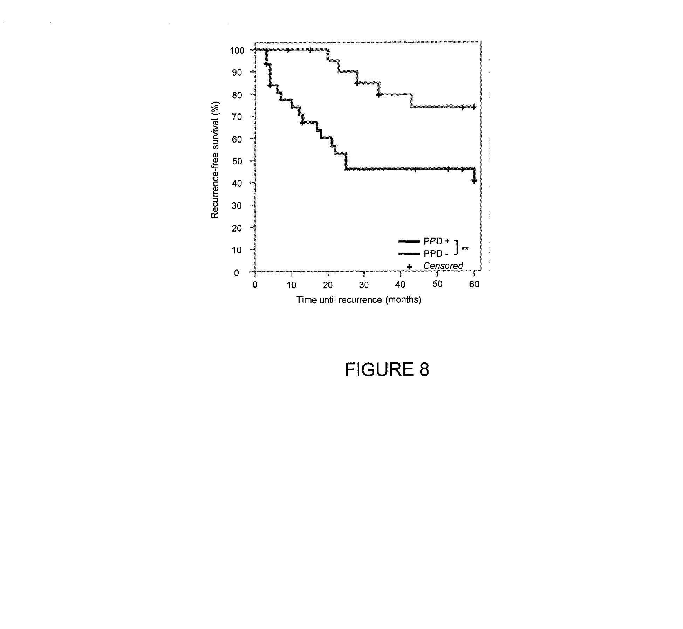

Using an experimental model, the inventors have demonstrated that BCG dissemination to bladder draining lymph nodes and priming of interferon-γ-producing T cells could occur following a single instillation. However, repeated instillations with live BCG were necessary for a robust T cell infiltration into the bladder. Subcutaneous immunization with BCG prior to instillation overcame this requirement, triggering a more robust acute inflammatory process following the first intravesical instillation and accelerating T cell entry into the bladder, as compared to the standard protocol. Moreover, subcutaneous immunization with BCG prior to intravesical treatment of an orthotopic tumor dramatically improved response to therapy (Figure 7). Importantly, retrospective analysis of clinical data, illustrated a similar finding: patients with immune signatures of prior exposure to BCG had a significantly better recurrence-free survival (Figure 8). Together these data suggest that monitoring patients' response to BCG (measurable for example by patient's response to purified protein derivative (PPD)), and, in their absence or weak level, priming or boosting BCG responses by parenteral exposure prior to intravesical treatment initiation, may be a safe and effective means of improving intravesical BCG-induced clinical responses.

These data thus provide critical new insight into a long-standing clinically effective immunotherapeutic regimen and suggest strategies that may improve patient management.

The inventors have demonstrated that inducing BCG-specific immunity prior to local therapy improves anti-tumor response. However, BCG-specific immunity can be induced, not only by immunization with BCG, but also by immunization with antigenically-related non-pathogenic mycobacteria or immunogenic component(s) thereof. For these reasons, like BCG, other antigenically-related non-pathogenic mycobacteria could be used for cancer immunotherapy.

In addition, the drastic anti-tumor response improvement observed by using BCG pre-immunization in bladder cancer treatment, suggest that an anti-tumor response could also be observed by using the same strategy for treating other cancers for which standard BCG immunotherapy may have failed thus far. Therefore, BCG pre-immunization should increase the number of cancer types that can be treated using BCG immunotherapy.

Therefore, the present invention relates to a first and a second identical or different mycobacterial immunogenic compositions, each comprising at least a *Mycobacterium bovis* bacillus Calmette-Guérin (BCG), an antigenically related non-pathogenic mycobacteria, or one or more immunogenic component(s) thereof, as therapeutic active ingredient(s) for use in the treatment of cancer by parenteral or oral administration of the first composition to a cancer patient before local administration of the second composition at tumor site.

According to the present invention, a non-pathogenic mycobacteria antigenically related to BCG refers to an avirulent or attenuated mycobacteria which induces a BCG-specific immune response. The tumor site refers to the site of tumors, before and after tumor resection.

The present invention encompasses the use of whole cell, live or killed, non-pathogenic mycobacteria. Non pathogenic mycobacteria include naturally avirulent *Mycobacterium species,* attenuated strains (genetically modified or not) of *Mycobacterium sp*., and recombinant strains derived from the preceding strains.

A mycobacteria for use in the present invention may be a recombinant BCG (rBCG) improved through addition of relevant genes such as Th1 cytokines (IL2, GM-CSF, IFN-γ, IFN-α2, IL-18, MCP-3, IL-15, TNF-α), BCG or *Mycobacterium tuberculosis* immunodominant antigens such as *Tuberculosis* Ag85B (rBCG30), or listeriolysin (rBCGΔureC:Hly or VPM1002; Kaufmann et al., Lancet, 2010, 375, 2110-2119). rBCG30 and VPM1002 are candidate vaccines for tuberculosis that have entered clinical trials. It may also be a recombinant mycobacteria expressing a mycobacterial FAP protein under the control of a promoter active under hypoxia conditions (International PCT Application WO 2008/012693).

Another mycobacteria for use in the present invention may be a genetically modified *M. tuberculosis* that has been attenuated through deletion of virulence genes such as *phoD and fadD26* (MTBVAC01; Kaufmann et al., Lancet, 2010, 375, 2110-2119).

Yet another mycobacteria for use in the present invention may be a naturally avirulent or attenuated mycobacteria such as *M. microti, M. smegmatis, M. fortuitum, M. vaccae, M. hiberniae, M. terrae, M. triviale, M. triplex, genavense, M. kubicae, M. heidelbergense, M. cookii, M. haemophylum, M. botniense, M. conspicuum, M. doricum, M. farcinogenes, M. heckeshornense, M. monacense, M. montefiorense, M. murale, M. nebraskense, M. saskatchewanense, M. scrofulaceum, M shimnodei, M tusciae, M xenopi, M chelonae, M. boletii, M peregrinum, M porcinum, M senegalense, M. houstonense, M mucogenicum, M mageritense, M austroafricanum, M. diernhoferi, M. hodleri, M frederiksbergense, M aurum, M chitae, M fallax, M confluentis, M flavescens, M madasgkariense, M phlei, M gadium, M. komossense, M obuense, M. sphagni, M. agri, M aichiense, M alvei, M arupense, M. brumae, M canariasense, M chubuense, M duvalii, M elephantis, M gilvum, M. hassiacum, M holsaticum, M immunogenum, M massiliense, M moriokaense, M. psychrotolerans, M pyrenivorans, M. vanbaalenii, M pulveris, M. arosiense, M aubagnense, M. chlorophenolicum, M. fluoranthenivorans, M. kumamotonense, M. novocastrense, M parmense, M. phocaicum, M poriferae, M. rhodesiae, M. seoulense, and M. tokaiense, and subspecies.*

BCG for use in the present invention is preferably a commercial available BCG strain which has been approved for use in humans such as Pasteur, Frappier, Connaught (Toronto), Tice (Chicago), RIVM, Danish 1331, Glaxo-1077, Tokyo-172 (Japan), Evans, Prague, Russia, China, Sweden, Birkhaugh, Moreau, and Phipps.

Killed mycobacteria, either killed but metabolically active or killed and metabolically inactive, are prepared according to methods well-known in the art which include treating mycobacteria with physical agents such as for example heat, UVA or gamma radiations and/or chemical agents such as formalin and psoralen. Killed but metabolically active mycobacteria refers to mycobacteria that are viable and able to express their genes, synthesize and secrete proteins but are not culturable *(i.e.,* not capable of colony formation) because they are not replicative. Killed but metabolically active mycobacteria include for example nucleotide excision repair mutants which have been inactivated by photochemical treatment with psoralen and/or UV light. Killed and metabolically inactive include for example gamma-irradiated mycobacteria, heat-killed mycobacteria and extended freeze-dried killed mycobacteria (International PCT Application WO 03/049752).

The present invention encompasses also the use of immunogenic components such as subcellular fractions and recombinant antigens (proteins and vectors encoding said proteins) from BCG or antigenically related non-pathogenic mycobacteria. Said immunogenic components are well-known in the art and include for example : (i) mycobacterial cell wall fraction, eventually complexed with DNA (MCC; Morales et al., J. Urol., 2009, 181, 1040-1045; Morales et al., J. Urol., 2001, 166, 1633-; Chin et al., J. Urol., 1996, 156, 1189-; Uenishi et al., Chemical and Pharmaceutical Bulletin, 2007, 55, 843-852; Azuma et al., J. Natl. Cancer Inst., 1974, 52, 95-; Takeya, K and Hisastsuna, K., J. Bacteriol., 1963, 85, 16-: Fox et al., J. Bacteriol., 1966, 92, 1-) or R8 liposome (Joraku et al., BJU Int., 2009, 103, 686-693) and (ii) *Tuberculosis* or BCG recombinant immunodominant antigens such as Ag85A, Ag85B, TB10.4, Mtb32, Mtb39, ESAT-6, used as fusion proteins consisting of one or more antigens, or expressed by a recombinant vector such as a replication-deficient vaccinia virus or E1-deleted adenovirus (Kaufmann et al., Lancet, 2010, 375, 2110-2119). The recombinant proteins are formulated in a vaccine adjuvant such as a mixture of oligodeoxynucleotides and polycationic amino acids or monophosphoryl lipid A and QS21.

In a particular embodiment of the present invention, the first composition comprises a live or killed but metabolically active non-pathogenic mycobacteria. Preferably, said composition is for parenteral or oral administration to a patient having no active immune response to BCG, as assessed by example by a weak positive or a negative PPD skin test. More preferably, the composition comprises a live non-pathogenic mycobacteria selected from the group consisting of: BCG, a rBCG expressing Th1 cytokines, BCG or *Tuberculosis* immunodominant antigens, or listeriolysin, and a genetically modified *M. tuberculosis* that has been attenuated through deletion of virulence genes.

In another particular embodiment of the present invention, the first composition comprises one or more immunogenic component(s) of the non-pathogenic mycobacteria. Preferably, said composition is for parenteral or oral administration to a patient having an active immune response to BCG, as assessed by example by a positive PPD skin test. More preferably, the composition comprisesone or more *M.tuberculosis* or BCG recombinant immunodominant antigens or recombinant vector(s) expressing said antigens, or a mycobacterial cell wall fraction.

The parenteral or oral administration is at any time after cancer diagnosis. It is usually before tumor resection but can be concomitant with tumor resection. It is preferably performed, just after the diagnosis. It may be subcutaneous (s.c.), percutaneous, intradermal,intramuscular or oral, more preferably subcutaneous (s.c.), percutaneous or intradermal. It usually comprises one single administration.

In another particular embodiment of the present invention, the second composition comprises live or killed (killed but metabolically active or killed and metabolically inactive) non-pathogenic mycobacteria or one or more immunogenic components thereof as defined above.

According to the present invention, the first and the second composition may be the same composition or different compositions. When different compositions comprising different mycobacteria or different immunogenic components are used for the parenteral or oral and the local administration, the mycobacteria or immunogenic components are chosen so that they have B, T CD4+ and/or T CD8+ epitopes in common. Using this type of compositions will ensure that the local administration will boost the specific immune response induced by the parenteral or oral administration.

The local administration is usually after tumor resection and at least seven days after the parenteral or oral administration. Preferably, it is at least three weeks after the parenteral or oral administration. The local administration at tumor site will depend on the type of cancer. For example, for bladder cancer it is intravesical. It usually comprises at least one series of at least three separate administrations, usually between three to six administrations, at an interval of one to three weeks. For the maintenance therapy, additional series of repeated administrations are generally performed using a similar administration regimen. The intravesical administration regimen recommended for BCG by the European and American guidelines comprises an induction course of 6 weekly intravesical instillations, followed by maintenance therapy. The recommended maintenance regimen consists in 3 weekly instillations at 3 months, 6 months, and then every 6 months up to 3 year. This recommended administration regimen can be used for the composition of the present invention in the treatment of bladder cancer.

The composition(s) for use in the present invention comprise a pharmaceutically effective dose of a non-pathogenic mycobacteria or one or more immunogenic component thereof. A pharmaceutically active dose is that dose required to prime or boost a BCG specific immune response in a patient and improve the anti-tumor response leading to a better recurrence-free survival compared to untreated patients or patients treated by local administration only. The pharmaceutically effective dose depends upon the composition used, the route of administration, the type of mammal (human or animal) being treated, the physical characteristics of the specific mammal under consideration, concurrent medication, and other factors, that those skilled in the medical arts will recognize. Generally, the dose of live non-pathogenic mycobacteria in the composition depends on the age of the patients. For human adults, it is in the range of 10⁷ to 10¹⁰ CFUs (Colony Forming Units) for the local and parenteral or oral administrations, preferably about 10⁸ to 10⁹ CFUs. The dose of killed non-pathogenic mycobacteria in the composition is in the range of 50 to 150 mg, which corresponds to the amount of killed mycobacteria obtained starting with 10⁹ to 10¹¹ CFUs before the killing. The dose of mycobacterial cell wall fraction is in the range of 1 to 10 mg, preferably formulated in an emulsion.

The composition(s) for use in the present invention may further comprise one or more additional agents like : (i) pro-inflammatory agents such as inflammatory cytokines (IL-2, IFN-α, TNF-α, GM-CSF), (ii) T-cell stimulatory molecules such as agonist antibodies directed against T-cell activating co-stimulatory molecules (CD28, CD40, OX40, GITR, CD137, CD27, HVEM) and blocking antibodies directed against T-cell negative co-stimulatory molecules (CTLA-4, PD-1, TIM-3, BTLA, VISTA, LAG-3), (iii) antibiotics, and (iv) chemotherapy drugs.

Alternatively, the composition (s) comprising a non-pathogenic mycobacteria or immunogenic component(s) thereof may be used in combination (separate or sequential use) with such additional agents.

For example, antibiotic(s) such as ofloxacin may be used in combination with the composition comprising live BCG or antigenically related non-pathogenic mycobacteria strain, to reduce side-effects in patients.

The composition(s) for use in the present invention usually comprises a pharmaceutically acceptable carrier. The composition is further formulated in a form suitable for parenteral, oral, and/or local (intravesical, intravaginal or epicutaneous) administration into a subject, for example a mammal, and in particular a human.

Examples of cancer that can be treated using the treatment of the invention include with no limitation: bladder, melanoma, cervical, colon, prostate, ovarian and breast cancer.

In another particular embodiment of the invention, the cancer is a mucosal cancer including with no-limitation, non-muscle invasive (Ta, carcinoma *in situ* (Tis), T1) and muscle invasive (T2, T3, T4) transitional cell carcinoma of the bladder, cervical and colon cancers.

Preferably said mucosal cancer is superficial or non-invasive, *i.e*., low-stage tumor. In a more preferred embodiment, said mucosal cancer is is a non-muscle invasive bladder cancer selected from the group consisting of: carcinoma *in situ* (Tis) and high-grade Ta or T1 transitional cell carcinoma of the bladder.

According to a first preferred embodiment of the invention, a first and a second composition comprising live BCG are used for the treatment of bladder cancer. Preferably, the first composition comprising 10⁷ to 10⁹ CFUs of BCG Pasteur or Danish strains is injected intradermally (ID) to a PPD negative patient, shortly after bladder cancer diagnosis. The second composition comprising 10⁷ to 10⁹ CFUs of BCG Connaught strain is then instilled intravesically after tumor resection, three weeks after the ID injection, using the intravesical administration regimen recommended for BCG by the European and American guidelines.

Another aspect of the present invention relates to a method *in vitro* for monitoring cancer treatment by immunotherapy with BCG or antigenically related non-pathogenic mycobacteria, comprising:
- assaying BCG-specific immune response in a sample from a patient, wherein a positive assay correlates with an active anti-tumor response in the patient.

BCG-specific immune response may be assayed by standard assays well-known in the art. For example, BCG-specific antibodies may be detected by ELISA, BCG-specific CD4+ T-cells and CD8+ T-cells may be detected by proliferation assays (CSFE assay), cytokine assays (ELISPOT, Intracellular cytokine staining) or immunolabeling assays (FACS assay). Antigens that can be used for assaying a BCG specific immune response are well-known in the art and include the purified protein derivative (PPD) from *M. tuberculosis* and isolated immunodominant antigens of BCG or antigenic fragments thereof comprising B, CD4+ or CD8+ T-cells epitopes. For example, BCG Antigen 85 may be used to detect a BCG specific immune response and the HLA-2 restricted peptides Ag85A(6-14) and Ag85A(200-208) may be used to detect CD8 specific responses.

The assay is performed on a biological material containing antibodies and T-cells. For example, it may be performed on a whole body fluid such as blood or urine, or on a fraction thereof.

For example, the QuantiFERON^{®}-TB test which is based on the quantification of interferon-gamma (IFN-γ) released from sensitized lymphocytes incubated overnight with purified protein derivative (PPD) from *M. tuberculosis* and control antigens can be used to assay BCG-specific T-cell response in patients.

The method may comprise the detection of antibodies, CD4+ T-cells, or CD8+ T-cells specific to BCG.

The assay is performed before to initiate the immunotherapy, as well as during the immunotherapy, to optimize the administration regimen and in turn improve the anti-tumor response in the patient.

According to another preferred embodiment, the BCG-specific immune response is assayed before to initiate the immunotherapy, in order to determine which composition should be administered by the parenteral route (live mycobacteria or subunit vaccine), and eventually, just before or after tumor resection, before the first local administration, and/or at the end of the local administrations of the BCG, antigenically related non-pathogenic mycobacteria, or immunogenic component(s) thereof at tumor site.

For a better understanding of the invention and to show how the same may be carried into effect, there will now be shown by way of example only, specific embodiments, methods and processes according to the present invention with reference to the accompanying drawings in which:

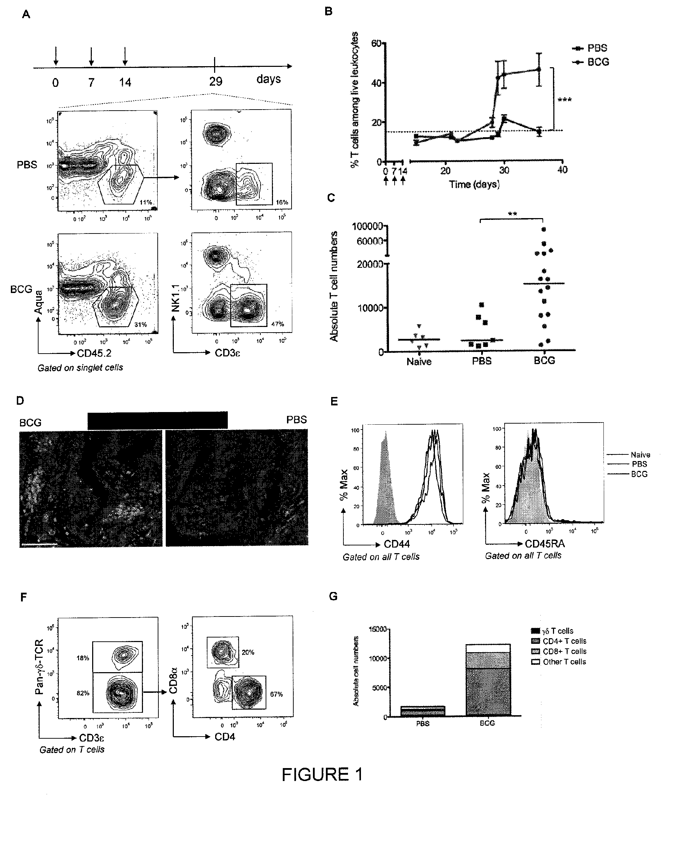

**Figure 1****.** Shows that repeated instillations of BCG result in a robust, though late, infiltration of activated αβ T cells into the bladder. (A) Female mice received 3 weekly intravesical instillations of PBS (control) or clinical-grade BCG (Immucyst) at days 0, 7, 14 (indicated by black arrows). At day 29, bladders were resected, digested with collagenase and stained for flow cytometry. T cells were gated as live CD45⁺ CD3ε⁺ NK1.1⁻ cells. Representative FACS plots are shown. (**B**) Mice were treated as above and the kinetics of T cell infiltration was evaluated. The dashed line indicates basal level T cells in naïve controls; independent experiments were combined with n=3-7 mice analyzed per time point; means and SEM are shown. A general linear mixed model was used to compare the percentage of T cells among total leukocytes (***, p<0.0001). (**C**) Data from (**B**) was re-analyzed and absolute T cell numbers are shown for individual mice during the time window of maximal infiltration (day 29 to 35). Black bars indicate median. A Mann-Whitney test was performed (**, p<0.01). (**D**) Immunofluorescence staining at day 33 is shown. Nuclei were stained with DAPI (grey), leukocytes with CD45.2 (green) and T cells with CD3ε (red), whereas α-smooth muscle actin (SMA, blue) staining indicates the smooth muscle layer of blood vessels (V) and the bladder muscle layer (M). A dotted white line demarcates the bladder lumen (L), the thin urothelial layer (U) and the submucosa (S) are indicated. Scale bar = 100µm. (**E**) Bladder infiltrating T cells were assessed by cytometry for an activated phenotype based on CD44 expression and absence of CD45RA. A representative histogram is shown. Shaded histograms indicate fluorescence minus one. (**F-G**) T cells infiltrating the bladder were further gated as γδ-TCR positive or negative; and the latter population was assessed for CD4 or CD8α expression. Representative FACS plots and gating strategy is shown (**F**). Average numbers of T cell subpopulations are displayed; n=4 mice per group (**G**).

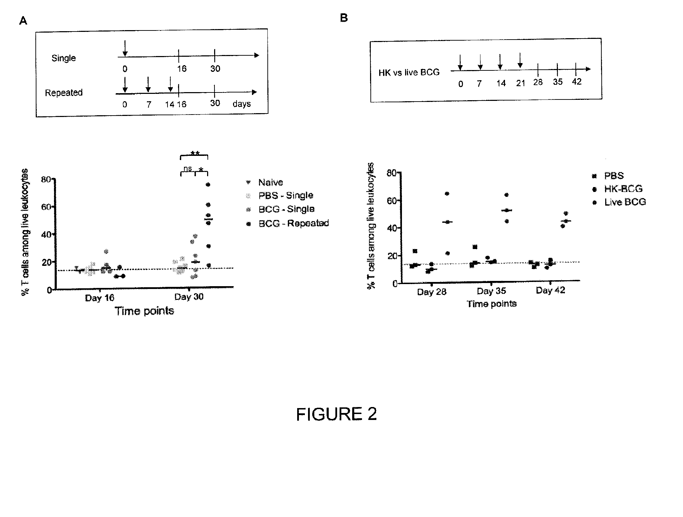

**Figure 2**. Shows that repeated instillations and live BCG are required, in order to achieve bladder T cell infiltration. (**A**) Mice received either a single instillation (PBS or BCG) or 3 weekly-repeated instillations (BCG) and at indicated time points, the frequency of T cells infiltrating the bladder was assessed by flow cytometry. Individual mice and medians are shown; the dashed line represents the basal level in naïve littermates. Mann-Whitney tests were performed (ns, non significant; *, p<0.05; **, p<0.01). (**B**) Mice received 4 weekly-repeated instillations of either PBS or live or heat-killed (HK) BCG and at indicated time points, the frequency of T cells infiltrating the bladder was assessed by flow cytometry. Individual mice and medians are shown; the dashed line represents the basal level in PBS-treated littermates.

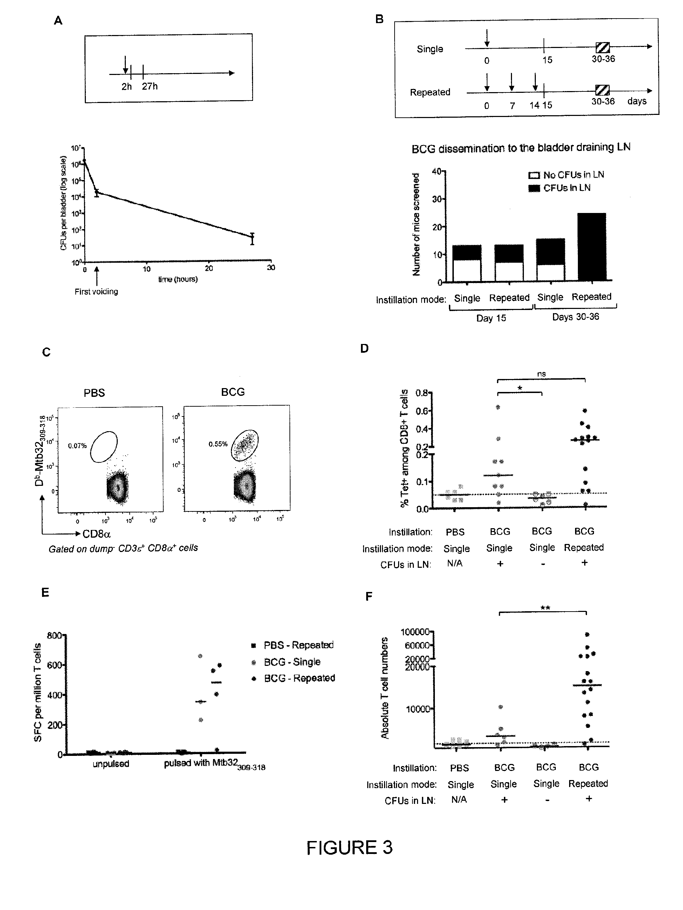

**Figure 3****.** Shows that priming of T cells and their entry into the bladder are temporarily disconnected following intravesical BCG regimen. (A) At 2 and 27 hours following instillation, bladders were homogenized in PBS and total CFUs per organ were enumerated. (**B**) Bladder draining LN were resected at indicated time points, after either a single or repeated instillation(s) of live BCG, homogenized in PBS and plated. Mice were stratified as either CFU positive (black) or CFU negative (white); several independent experiments were combined (*n* = 13-24 mice per group). (**C-D**) Mice were treated and stratified as above and the BCG-specific response was analyzed on splenocytes using H2-D^{b}-Mtb32₃₀₉₋₃₁₈ tetramers on day 30-36. CD8⁺ T cells were gated as live, dump negative (dump channel including CD45RB (B220), NK1.1, CD11b, F4/80 and CD4), CD3ε⁺ CD8α⁺ and the percentage of tetramer positive cells among this population was analyzed. A representative FACS plot for tetramer assays is shown for an animal receiving PBS or weekly intravesical instillations of BCG (**C**). The percentage of tetramer positive (Tet+) cells among CD8+ T splenocytes is shown for individual mice across the different treatment conditions and black bars represent medians. Mann-Whitney tests were performed (ns, non significant; *, p<0.05) (**D**). (**E**) Mice were treated as above, and at day 29, purified CD8+ T cells from spleen and draining LN from mice that were CFU⁺ were restimulated *ex vivo* for 20h using splenocytes pulsed with Mtb32₃₀₉₋₃₁₈ peptide. Unpulsed splenocytes served as a negative control. The number of spot forming cells (SFC) per 10⁶ CD8⁺ T cells for individual mice is shown. (**F**) Mice were treated and stratified as above and absolute numbers of T cells infiltrating the bladder were enumerated following either a single or repeated instillation(s) on day 30-36. Individual mice are shown; black bars represent medians. Mann-Whitney tests were performed (**, p<0.01).

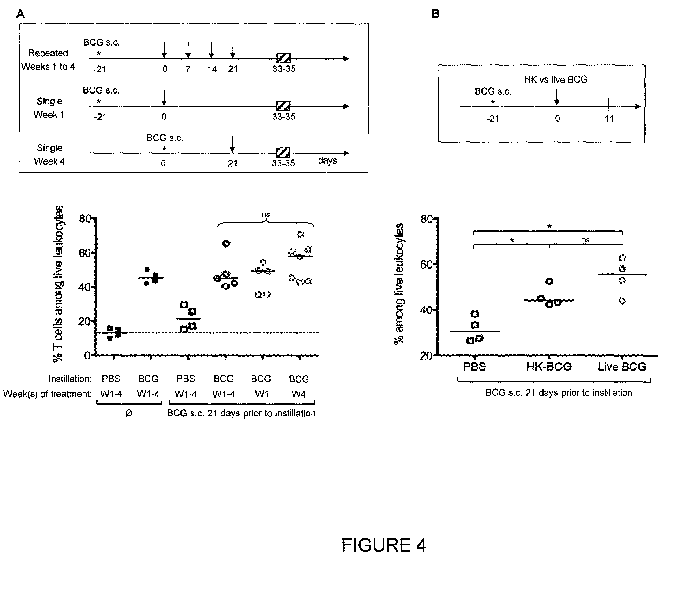

**Figure 4****.** Shows that subcutaneous immunization with BCG prior to intravesical instillation(s) results in accelerated T cell entry into the bladder, following intravesical challenge with live or heat-killed (HK) BCG, thus overcoming the requirement for repeated instillations. **(A)** Twenty-one days prior to intravesical instillation, mice were subcutaneously (s.c.) immunized with BCG, as compared to non-immunized (Ø) controls (s.c. injection is represented by a star). Mice subsequently received either a single or repeated intravesical instillation(s) with PBS or BCG (instillations represented by a black arrow). Bladder T cell infiltration was analyzed by flow cytometry on day 33-35. A Kruskal-Wallis test was performed among all groups that received intravesical BCG (ns, non significant). (**B**) Twenty-one days post s.c. immunization, mice received a single intravesical instillation with PBS, live or HK BCG and bladder T cell infiltration was assessed by flow cytometry on day 11. Individual mice and medians are shown. Mann-Whitney tests were performed (ns: non significant; * p<0.05).

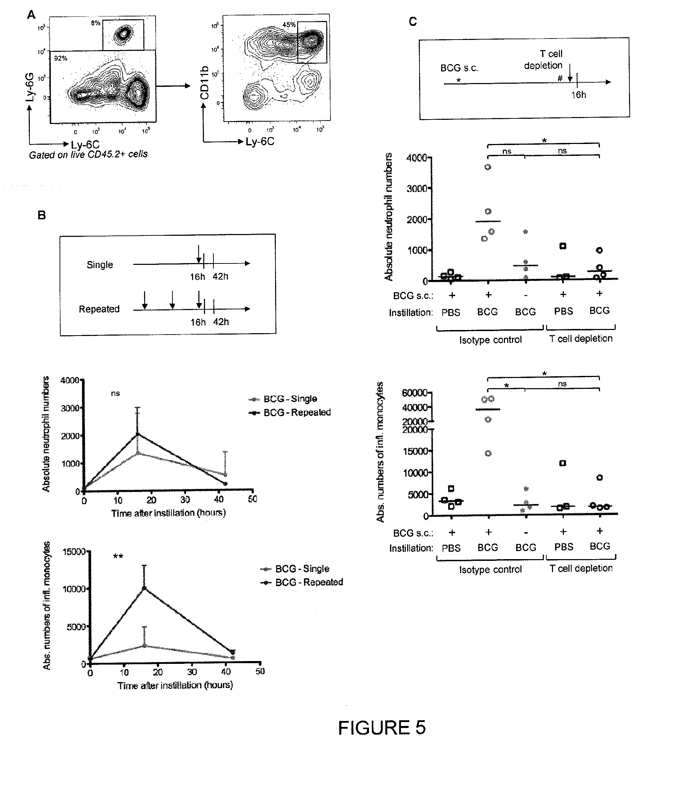

**Figure 5****.** Shows that pre-existing adaptive immunity supports a robust, albeit short-lived innate immune response. (**A**) Neutrophils were defined as live CD45.2⁺ Ly-6G⁺ cells; inflammatory monocytes were defined as live CD45.2⁺ Ly-6G⁻ Ly-6C^{high} CD11b⁺ cells. For each cell population, a representative FACS plot is shown (sixteen hours after third BCG instillation). (**B**) Sixteen and forty-two hours following either the first or third BCG instillation, bladder-infiltrating neutrophils (upper graph) and inflammatory monocytes (lower graph) were quantified by flow cytometry (*n* = 3-9 mice per group). Mean values and SEM are shown. A Mann-Whitney analysis was performed to compare infiltration at sixteen hours following the first and the third instillation with BCG (ns, non significant; ** p<0.01). (**C**) Mice were s.c. immunized with BCG twenty-one days prior to instillation, as compared to non-immunized controls, followed by a single intravesical instillation with PBS or BCG. Forty-eight hours prior to instillation, mice were treated with depleting monoclonal antibodies specific for CD4⁺ and CD8⁺ T cells or isotype control antibodies. Sixteen hours after intravesical instillation, infiltration of neutrophils (upper graph) and inflammatory monocytes (lower graph) was assessed by flow cytometry. Individual mice are shown and medians are indicated by black bars. Mann-Whitney analyses were performed (ns, non significant; * p<0.05).

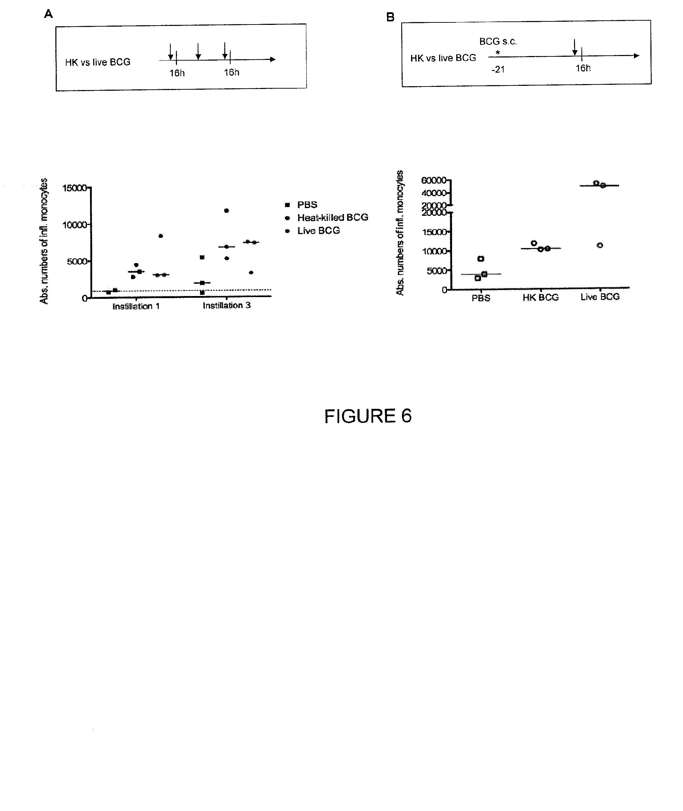

**Figure 6****.** Shows that Intravesical HK-BCG triggers a similar inflammatory response in the bladder.(A-B) Sixteen hours after the instillation of interest, bladders were resected and analyzed as described above. Total numbers of inflammatory monocytes for individual mice are shown here; medians are indicated by a black line. Mice received either 3 weekly instillations of PBS, live or HK BCG (A) or mice were immunized s.c. with BCG and 21 days later, they received a single instillation of PBS, live or HK BCG (B).
**Figure 7****.** Shows that pre-existing BCG-specific immunity improves anti-tumor response in a mouse model for bladder cancer. Three weeks prior to orthotopic MB49 tumor challenge, mice were s.c. immunized with BCG (solid lines) or left untreated (dashed lines). Starting two days after tumor challenge, mice received 5 weekly intravesical instillations of either PBS (blue lines) or BCG (red lines) and were monitored twice daily for survival until termination of the experiment on day 70. A log-rank test was performed to compare groups that received intravesical BCG, either immunized s.c. or not (** p<0.01).
**Figure 8****.** Shows that pre-existing BCG-specific immunity improves the anti-tumor response in patients with high-risk non-muscle invasive bladder cancer undergoing intravesical BCG therapy. Patients were stratified according to their pre-therapy purified protein derivative (PPD) status (+, positive; -, negative), and a retrospective analysis of their recurrence-free survival was performed over 60 months. The median recurrence-free survival was 25 months in the PPD negative group and not reached in the PPD positive group. A log-rank test was performed (** p<0.01); hash marks along the lines indicate censored events (e.g., death from causes other than bladder cancer).

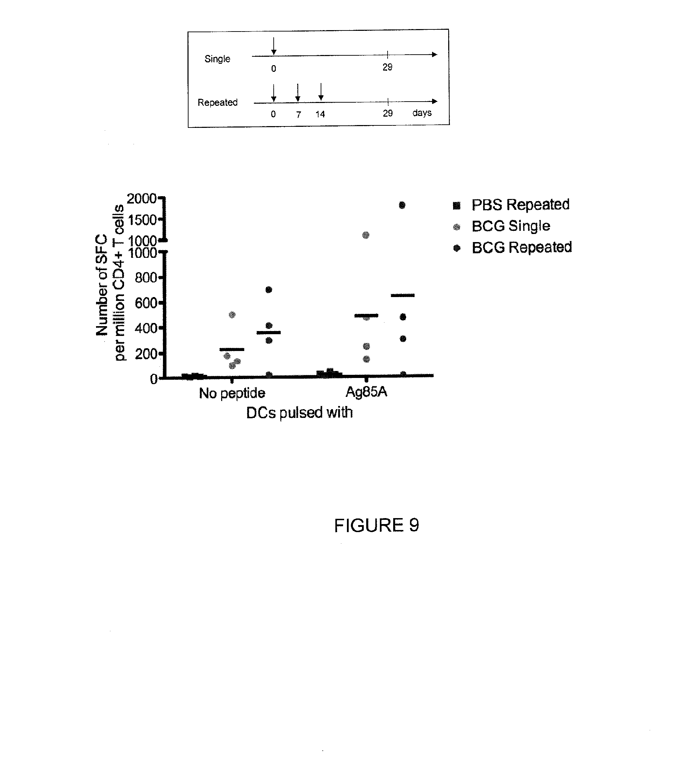

**Figure 9****.** Shows monitoring of BCG-specific T cell response following intravesical instillation with BCG. Mice received either a single or repeated intravesical instillation(s) with PBS or BCG, and at day 29, purified CD4⁺ T cells from spleen and draining lymph nodes were restimulated *ex vivo* for 20h using splenocytes pulsed with Ag85A₂₄₁₋₂₆₀ peptide. Unpulsed splenocytes served as a negative control. The number of spot forming cells (SFC) per 10⁶ CD4⁺ T cells for individual mice is shown.

There will now be described by way of example a specific mode contemplated by the Inventors. In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

### Example 1: Materials and methods

### 1.1 Mouse intravesical instillations and subcutaneous immunization

For intravesical instillations, 7-12 week-old C57BL/6 female mice (Charles Rivers) were water starved for 7-8 hours, reflecting the clinical practice of patients being asked not to drink prior to treatment. Mice were anesthetized (125 mg/kg ketamine and 12.5 mg/kg xylazine intraperitoneally) and drained of any urine present by application of slight digital pressure to the lower abdomen. The urethral orifice was disinfected with povidone-iodine and a 24Ga-catheter (BD Insyte Autoguard, Becton Dickinson) adapted to a 1mL tuberculin syringe (Braun) containing 50µL of either phosphate-buffered saline (PBS, Invitrogen) or BCG (about 3x10⁶ CFUs) was carefully inserted through the urethra. The injection was made at a low rate to avoid trauma and vesico-ureteral reflux, and there was no dead volume in the catheter. Mice were kept under anesthesia for 2 hours, with catheter and syringe maintained in place to retain the intravesical solution. For tumor challenge, mouse bladders were pre-treated with 0.1mg/mL poly-L-lysin (Sigma-Aldrich) for 20 minutes, prior to instillation of 80,000 MB49 cells in 50µL PBS, which were retained for 1 hour into the bladder. For subcutaneous (s.c.) immunization, mice received a single injection of 2-5x10⁶ CFUs BCG. Mice were housed under specific-pathogen free conditions and used under approved protocols.

### 1.2 BCG and determination of bacterial load

For instillations, Immucyst (Sanofi Pasteur) was reconstituted in 3mL PBS following the manufacturer's instructions. Heat-killed BCG was obtained by autoclaving Immucyst preparation 20 min at 121°C. For s.c. administration, either Immucyst (once) or frozen aliquots of BCG Pasteur (1137P2) were used with similar results. BCG-Pasteur was grown at 37°C in Middlebrook 7H9 medium supplemented with bovine albumin, dextrose and catalase (ADC, Difco), harvested in exponential growth phase, washed, dispersed with 3mm glass-beads, resuspended in PBS, aliquoted and then frozen at -80°C. A defrosted aliquot was used to determine the lot titer on 7H11 medium supplemented with oleic acid, albumin dextrose and catalase (OADC, Difco). In addition, all preparations used for intravesical or subcutaneous injections were titrated. For organ bacterial load, bladders were resected in sterile PBS, homogenized 2 min at 25Hz in a Tissue Lyzer II (Qiagen) while draining lymph nodes (LN) were mashed with the back of a syringe in sterile PBS. Five-fold serial dilutions of the homogenates were plated on 7H11 supplemented with OADC and colony forming units (CFUs) were assessed after 17-28 days of growth at 37°C.

### 1.3 Antibodies and reagents

For FACS, CD16/CD32 (clone 2.4G2, Fc block), CD45.2 (clone 104), CD3ε (clone 145-2C11), NK1.1 (clone PK136), CD8α (clone 53-6.7), CD44 (clone IM7), CD45RA (clone 14.8), CD45R/B220 (clone RA3-6B2), CD11c (clone HL3), CD86 (clone GL1), Ly-6C (clone AL-21), Ly-6G (clone 1A8) antibodies (Abs) were purchased from BD Pharmingen; CD4 (clone GK1.5), CD11b (clone MAC-1), pan-γδ TCR (clone GL3), IA^{b}-IE^{b} (clone M5), F4/80 (clone BM8) Abs were from eBioscience and CD45.2 (clone 104-2) from Southern Biotech. Dead cells were stained either with 4',6-diamidino-2-phenylindole (DAPI, Sigma-Aldrich) or with live/dead fixable Aqua dead cell staining kit (Invitrogen). Cells were enumerated using Accucheck counting beads (Invitrogen). For histology, CD3ε (clone 500A2) and CD45.2 (clone 104) Abs were obtained from BD Pharmingen; α-smooth muscle actin (α-SMA, clone 1A4) from Sigma-Aldrich and syrian-Hamster secondary Ab from Jackson ImmunoResearch Laboratories. Abs used in the IFN-γ ELISPOT assays were purchased from Mabtech. H2-D^{b}-restricted Mtb32₃₀₉₋₃₁₈ peptide (GAPINSATAM) an I-A^{b} Ag85A₂₄₁₋₂₆₀ peptide (QDAYNAGGGHNGVFDFPDSG) were obtained from PolyPeptide. Depleting anti-CD4 (clone GK1.5) and anti-CD8 (clone YTS169.4) as well as rat IgG2b isotype control mAbs were purchased from Bio X Cell. MB49 cells were received from the Brandau group and cultured in D-MEM (Invitrogen), complemented with 10% fetal calf serum (FCS, Eurobio) and 1% Penicilin/Streptomycin (Invitrogen). Poly-L-lysin was purchased from Sigma-Aldrich.

### 1.4 Tissue processing and flow cytometry

Bladders were resected and incubated in DMEM (Invitrogen) containing 1mg/mL collagenase D (Roche), 0.17U/mL liberase TM (Roche) and 1U/mL Deoxyribonuclease 1 (Invitrogen) at 37°C for two successive cycles of 30 min. Tissue suspensions were washed in DMEM + 10% FCS, pressed through a 70-µm mesh, washed in PBS + 2% FCS, pressed through a 40-µm mesh and pelleted for FACS staining. Spleens were mashed, incubated at 37°C in 1.66% ammonium chloride (VWR International) in water for 5 min for red blood cell lysis and filtered through a 70-µm mesh. All cells were preincubated with Fc block (and Aqua, if used), washed and incubated with appropriate Abs for 20 min in PBS + 0.5% FCS. Samples were run on a BD FACSCantoII cytometer (BD Biosciences) and analyzed using FlowJo (Treestar) software.

### 1.5 Monitoring BCG-specific T cell response

BCG-specific T cell responses were tested by IFN-γ ELISPOT assays. For IFN-γ ELISPOT assays with CD4+ or CD8+ T cells, at indicated time points, spleens and bladder draining LN were harvested and combined, CD4+ and CD8⁺ T were purified using microbeads and MS columns (Miltenyi Biotec) and ELISPOT assays for IFN-γ-producing cells were performed as previously described (Blachere et al., PLoS. Biol., 2005, 3, e185).

In particular, for IFN-γ ELISPOT assays with CD4+ T cells, purified CD4⁺ T cells from spleen and draining lymph nodes were restimulated *ex vivo* for 20h using splenocytes pulsed with Ag85A₂₄₁₋₂₆₀ peptide. Unpulsed splenocytes served as a negative control. The number of spot forming cells (SFC) per 10⁶ CD4⁺ T cells was then determined for individual mice.

For IFN-γ ELISPOT assays with CD8+ T cells, , purified CD8+ T cells from spleen and draining LN were restimulated *ex vivo* for 20h using splenocytes pulsed with Mtb32₃₀₉₋₃₁₈ peptide. Unpulsed splenocytes served as a negative control. The number of spot forming cells (SFC) per 10⁶ CD8⁺ T cells was then determined for individual mice.

The ELISPOT plate evaluation was performed in a blinded fashion by an independent evaluation service (Zellnet Consulting).

For tetramer staining, soluble D^{b}-Mtb32₃₀₉₋₃₁₈ monomers were produced using a modified version of that described (Bousso et al., Immunity, 1998, 9, 169-) and conjugated using premium grade streptavidin-PE (Invitrogen), added for 1 hour at room temperature.

### 1.6 Immunofluorescence histology

Tissues were processed as previously described (Peduto et al., J. Immunol., 2009, 182, 5789-). Briefly, samples were fixed overnight at 4°C in a fresh solution of 4% paraformaldehyde (Sigma-Aldrich) in PBS, embedded in OCT compound (Sakura Finetek) and frozen at -80°C. Frozen blocs were cut at 8-µm thickness and sections collected onto Superfrost Plus slides (VWR International). Slides were dried one hour and processed for staining or stored at -80°C. For staining, slides were first hydrated in PBS-XG (PBS containing 0.1% Triton X-100 (Sigma-Aldrich) and 1% FCS) for 5 min and blocked with 10% FCS in PBS-XG for 1 hour at room temperature. Slides were then incubated with primary antibodies in PBS-XG overnight at 4°C, washed, incubated with secondary antibodies for 1 hour at room temperature, incubated with DAPI for 5 min at room temperature, washed and mounted with Fluoromount-G (Southern Biotech). Slides were examined under an Axiolmager M1 fluorescence microscope (Zeiss) equipped with a CCD camera and images were processed with AxioVision software (Zeiss).

### 1.7 T cell depletion

Mice were injected intraperitoneally with a mixture of 100ug anti-CD4 and 100ug anti-CD8 antibody, or with 200ug isotype control, 48hrs prior to instillation. Depletion efficiency was controlled on blood and splenocytes.

### 1.8 Patients

Fifty-five patients with non-muscle invasive bladder cancer and previously treated with BCG were retrospectively evaluated. Patients had been tested for their purified protein derivative (PPD) status prior to therapy as a means of assessing potential adverse effects (e.g., allergic response to BCG). All patients had been treated by transurethral resection and were eligible for BCG therapy (tumor stage and grade is reported in Table 1, and subsequently received weekly instillations of BCG. Patients were followed according to clinical guidelines and tumor recurrence was defined based on biopsy or urine cytology. Kaplan-Meier curves indicate recurrence-free survival. Collection and use of clinical data was in accordance with the host institutional ethical review board and all patients provided informed consent.

### 1.9 Statistics

Unless otherwise indicated, two-tailed Mann-Whitney non-parametric tests were employed for statistical analyses using Prism software (Graphpad). Differences with ap value of 0.05 or less were considered significant. For the mouse tumor challenge (Figure 7), a log-rank test was performed. For patient data analysis (Figure 8), a log-rank test was performed using SPSS 18.0 (SPSS Inc.). For kinetic studies (Figure 1B), a general linear mixed model using Stata 11.0 (Stata Corporation) was employed.

### Example 2: Repeated intravesical instillations of BCG result in a robust but late infiltration of activated αβ T cells into the bladder

Based on the clinical practice of resecting the tumor shortly prior to adjuvant BCG therapy, the inventors began their studies in tumor-free mice. Therefore, to determine the dynamics of T cell infiltration into the bladder, age-matched female C57BL/6 mice were intravesically instilled with either phosphate-buffered saline (PBS; control) or clinical-grade BCG (Immucyst, Sanofi-Pasteur) once a week for a total of three instillations (Figure 1A, instillations indicated by black arrow). At defined time points, bladders were resected, digested as detailed in the materials and methods, and stained for cytometric analysis. Infiltrating T cells were defined as CD45.2⁺CD3ε⁺NK1.1⁻ cells (Figure 1A). Twenty-nine days after the start of the treatment, there was a robust increase in both the percentage of T cells among total leukocytes infiltrating the bladder (Figure 1B) and their absolute number (Figure 1C). Once established, this infiltration was sustained in the absence of additional treatments for greater than 10 days (Figure 1B). Additionally, the inventors demonstrated that administration of a fourth weekly instillation did not alter the kinetics of T cell influx into the bladder. Bladder T cells were predominantly found within the submucosa in the vicinity of blood vessels, with some having infiltrated the urothelium (Figure 1D). All bladder T cells had an antigen-experienced phenotype, based on expression of CD44 and absence of CD45RA (Figure 1E). That said it should be noted that, while fewer in number, resident T cells were also CD45RA⁻ CD44^{hi}, suggesting that entry into the submucosa was restricted to previously activated T cells. Phenotypic assessment demonstrated that greater than 70% of the T cells were αβ CD4⁺ and CD8⁺ T cells (Figure 1F-G).

Early attempts to use BCG as an anti-cancer agent have reported anti-tumor activity after a single intratumoral injection with BCG (Zbar et al., J. Natl. Cancer Inst., 1971, 46, 831-). However, the inventors observed that T cell frequency in the bladder never increased much above the basal level following a single instillation of BCG (Figure 2A).

### Example 3: Priming of T cells and their entry into the bladder uncoupled following intravesical BCG regimen

Interestingly, early clinical investigation in humans suggested that live BCG was required in order to achieve tumor immunity (Kelley et al., J. Urol., 1985, 134, 48-; Zbar et al., Natl. Cancer Inst. Monogr., 1972, 35, 341-), despite the fact that the clinical-grade lyophilized preparation contains only 5-10% live organisms (Behr M.A., Lancet Infect. Dis., 2002, 2, 86-). However, mechanistic reasons for the failure of heat-killed organisms to provoke a response remain unclear. To evaluate the possibility that T cell recruitment was dependant on live bacilli, repeated intravesical instillations of clinical-grade BCG (containing live BCG) *versus* heat-killed (HK) BCG was compared. As expected, the latter did not result in T cell recruitment to the bladder (figure 2B).

To assess the requirement for live BCG to activate adaptive immunity components, the inventors have evaluated BCG dissemination with the hypothesis that its entry into the bladder draining LN is a pre-requisite for priming and subsequent T cell infiltration of the bladder. Such a requirement has indeed been well documented in the context of low-dose *Mtb* lung infection (Wolf et al., J. Exp. Med., 2008, 205, 105-; Reiley et al., Proc. Natl. Acad. Sci. U S A, 2008, 105, 10961- ; Chackerian et al., Infect. Immun., 2002, 70, 4501-). They first assessed decay of BCG after intravesical injection, assaying colony-forming units (CFUs) that remain in the bladder after first voiding (at removal of catheter) at 2 hours, and on day 1 post-instillation. Consistent with what has been suggested for human treatments (Durek et al., J. Urol., 165, 2001, 1765-; Siatelis et al., J. Clin. Microbiol., 2011, 49, 1206-), the inventors demonstrated that the BCG load in the bladder rapidly decreased to 1% of the instilled dose and was barely detectable by 24h post-instillation (Figure 3A). Next, they investigated the presence of live BCG in the peri-aortic draining LN. As an additional parameter they tested single *versus* weekly-repeated instillation(s), as the rapid decay of BCG load in the bladder led them to hypothesize that repeated doses of live BCG might be required to result in efficient BCG dissemination to the draining LN. Mice were intravesically instilled with live BCG and the peri-aortic LN were homogenized and plated at defined time points. Analysis of early time points (hours) after a single instillation demonstrated no bacterial growth, thus indicating that bacilli were not tracking to the LN due to passive processes (e.g., resulting from potential trauma and/or anti-grade pressure during the instillations). Mice were tested for up to 1 month following single or repeated BCG instillation(s) and when bacterial growth was observed, the total CFUs per LN ranged from 8 to 1,700 colonies (median CFU = 50). Due to the high variance, the inventors scored animals as positive or negative for the presence of live BCG in the LN. Overall, they observed that 40-60% mice harbored BCG in their peri-aortic LN after a single intravesical instillation - this was consistent across a time course of 15 - 36 days (Figure 3B). In comparison, mice receiving multiple instillations also showed a mixed response at 15 days, but by day 30-36, BCG could be cultivated from the peri-aortic LN of all mice (Figure 3B).

The inventors next evaluated the priming of BCG peptide-specific T cells, assessed using H2-D^{b}-Mtb32₃₀₉₋₃₁₈ tetramers (also known as PepA or GAP; Irwin et al., Infect. Immun., 2005, 73, 5809-) (Figure 3C). Interestingly, when mice were stratified based on the presence of live BCG in their peri-aortic LN, they found that the majority of CFU⁺ animals possessed a high frequency of BCG-specific CD8⁺ T cells among total splenocytes. In contrast, there was no expansion of D^{b}- Mtb32₃₀₉₋₃₁₈ reactive T cells in mice for which live BCG was undetectable (Figure 3D). When comparing mice that had received single or repeated instillations, the critical parameter was the presence of live BCG (Figure 3D). they next assessed the capacity of CD8⁺ T cells purified from spleen and peri-aortic LN to produce IFN-γ upon restimulation with Mtb32₃₀₉₋₃₁₈ peptide in an ELISPOT assay. In mice harboring live BCG within their LNs, they found similar numbers of spot forming cells (SFCs) irrespective of the number of instillations (Figure 3E). These data demonstrate that the priming of IFN-γ producing BCG-specific T cells can occur following a single instillation and correlates with BCG dissemination to the bladder draining LN.

To investigate if dissemination of BCG also correlated with local adaptive immunity, the inventors examined lymphocyte populations in the bladder. While they observed low levels of T cell infiltration in CFU⁺ animals, the level of infiltration was significantly lower in mice that had received single *versus* repeated instillations (Figure 3F). Together these data suggest that priming of T cells may be uncoupled from their accumulation in the bladder.

### Example 4: Subcutaneous immunization with BCG prior to intravesical regimen results in faster T cell entry into the bladder following intravesical challenge with live or HK-BCG and overcomes the requirements for repeated installations and intravesical BCG to be alive

To further test the dissociation of priming from T cell trafficking, the inventors evaluated whether the activation of BCG-specific T cells prior to bladder instillations would impact T cell recruitment during the first intravesical instillation. Mice were injected subcutaneously (s.c.) with BCG, and after 21 days, intravesical instillations were initiated - comparing single vs. repeated BCG challenge. In mice primed by s.c. BCG, they observed a robust T cell infiltration as early as 12 days following a single instillation (Figure 4A, s.c. - BCG W4), which lasted up to 35 days post instillation (Figure 4A, s.c. - BCG W1). Of note, the level of T cell accumulation in the bladder was similar to that achieved by multiple intravesical treatments (Figure 4A, BCG W1-4). Interestingly, repeated instillations in the s.c. primed group (Figure 4A, s.c. - BCG W1-4) did not result in an enhanced accumulation of T cells as compared to other treatment conditions, suggesting that maximal intravesical responses can be achieved by a s.c. injection of BCG followed by a single instillation of BCG. They next asked if s.c. immunization with live BCG prior to bladder instillations could overcome the requirement for live BCG in the intravesical challenge. As shown, this was the case as intravesical HK-BCG resulted in a significant recruitment of T cells in mice previously immunized s.c. with live BCG (Figure 4B, comparison to PBS, *p* < 0.05).

### Example 5: Pre-existing adaptive immunity supports a robust, albeit short-lived, intravesical innate immune response

To further characterize the differential bladder T cell trafficking following different BCG regimens, the inventors evaluated the local inflammation of the bladder mucosa. Shortly after the first and the third instillation, they observed a rapid but short-lived (less than 42 hours post instillation) influx of neutrophils (characterized as Ly-6G⁺ leukocytes, Figure 5A-B) and inflammatory monocytes (characterized as Ly-6C^{high} CD11b⁺ Ly-6G⁻ leukocytes, Figure 5A-B). Notably, accumulation of inflammatory monocytes was significantly more pronounced after the third instillation (Figure 5B). Interestingly, in animals that had received prior s.c. BCG, the infiltration of neutrophils and inflammatory monocytes after a single dose of intravesical BCG was more pronounced than in non-vaccinated animals (Figure 5C, isotype control). The inflammatory response was stronger than that observed following repeated instillations with no prior s.c. exposure to BCG (Figure 5B-C).

Given the robust inflammatory process observed in mice immunized s.c. with BCG, the inventors hypothesized that the existence of BCG-specific T cells at the time of instillation was impacting upon the acute inflammatory process. To test this possibility, mice previously immunized s.c. with BCG were subjected to anti-CD4 and anti-CD8 depleting antibodies 48h prior to intravesical instillation. Following T cell depletion, they demonstrated a decrease in the number of neutrophils and inflammatory monocytes infiltrating the bladder (Figure 5C). Interestingly, the level of the inflammatory response in the group of mice that underwent transient depletion was in the range of what is observed following the first instillation with no prior s.c. BCG exposure (Figure 5C); these data suggest that T cell priming, achieved by s.c. BCG, mediate the 'boosted' inflammatory response following intravesical BCG.

Repeated intravesical instillations with HK-BCG do not result in T cell recruitment to the bladder. In a preliminary experiment, the inventors wondered if this could be due to a different inflammatory response, but the influx of inflammatory monocytes was found to be similar after the first and the third instillation with either live (clinical-grade) or HK-BCG (Figure 6A). Following s.c. immunization with live BCG, they could show that a single intravesical instillation with HK-BCG resulted in a rather high inflammatory response, as assessed by the number of inflammatory monocytes infiltrating the bladder shortly after instillation (Figure 6B). The level of the response however seemed lower than that obtained with clinical-grade BCG, but whether this is really significant is unclear as, the experiment was done only once with 3 mice per group. Based on these results, together with data presented in Figure 2B, they suggest that the failure to achieve T cell infiltration of the bladder following intravesical HK-BCG is due to a lack of T cell priming. Indeed, the activation of a local intravesical innate immune response during HK-BCG challenge is sufficient to attract previously primed T cells.

### Example 6: Pre-existing BCG-specific immunity improves anti-tumor response

Based on the ability to achieve stronger inflammation and earlier T cell recruitment to the bladder microenvironment, the inventors reasoned that s.c. exposure to BCG prior to intravesical BCG therapy might improve the anti-tumor response. To test that hypothesis they employed an orthotopic tumor model - implantation of syngeneic MB49 tumor cells into the bladders of C57/BL6 mice. Although the derived epithelial MB49 tumors grow in an aggressive manner (Chan et al., B.J.U. Int., 2009, 104, 1286-), this model remains, to their knowledge, the only mouse model in which intravesical BCG treatment has been shown to induce anti-tumor responses, when initiated 1-2 days after tumor implantation (Gunther et al., Cancer Res., 1999, 59, 2834-). To evaluate the impact of pre-existing BCG-specific T cells, mice were immunized s.c. with BCG and, after 3 weeks, 80,000 MB49 cells were implanted into the bladder mucosa, as described in the materials and methods. Two days later, intravesical BCG therapy was initiated, and mice were monitored twice daily for survival. Strikingly, 100% of mice that received BCG s.c prior to intravesical therapy survived as late as 70 days post tumor challenge; in comparison, 80% of mice with no prior BCG immunization succumbed within 50 days, despite intravesical BCG therapy (Figure 5). As a control, mice received BCG s.c., were challenged with tumors and received intravesical PBS, showing no evidence of delayed tumor growth (Figure 7).

These results prompted them to investigate the relevance of pre-existing BCG-specific immunity in patients with high-risk non-muscle invasive bladder cancer undergoing BCG therapy. Retrospective analysis of clinical data was performed, in which patients underwent a purified protein derivative (PPD) skin test prior to intravesical therapy (Figure 8 and Table 1).

**Table 1: Patients and tumor characteristics**

| | | |
|---|---|---|
| **Patients (n)** | 55 | |
| **Pre-treatment PPD status (n, %)** | | |
| **Positive** | 23 | 42% |
| **Negative** | 32 | 58% |
| **Gender (n, %)** | | |
| **Male** | 49 | 89% |
| **Female** | 6 | 11% |
| **Age at last surgery before BCG (yr)** | | |
| **Median** | 71 | |
| **Range** | 46-90 | |
| **Tumor characteristics* (n, %)** | | |
| **Ta grade 1/2, recurrent** | 12 | 22% |
| **Ta grade 3** | 4 | 7% |
| **T1 grade 2** | 6 | 11% |
| **T1 grade 3** | 25 | 45% |
| **CIS alone** | 8 | 15% |
| **CIS concomitant** | 14 | 25% |

| | | |
|---|---|---|
| *Ta/T1 indicate papillary tumors. CIS : carcinoma in situ | | |

A positive skin test is the signature of previous exposure and active immune response to BCG, *M. tuberculosis* or other mycobacteria. The inventors therefore stratified patient outcome data according to their PPD status prior to treatment, and observed that patients with a positive PPD had a significantly better recurrence-free survival than patients with a negative PPD skin test (Figure 6). Together these data suggest that boosting BCG-specific immunity prior to intravesical therapy might improve clinical response and tumor immunity.

### Example 7: Monitoring of BCG-specific T cell response following intravesical instillation with BCG

The BCG-specific CD4⁺ T cell response in mice that had received single or repeated intravesical instillation with BCG was assessed using IFN-γ ELISPOT assay (figure 9).

### Conclusions

While prior efforts have evaluated immunologic response during therapy in human observational studies or in experimental mouse models, the inventors study provides the first systematic evaluation of BCG-induced T cell infiltration of the bladder mucosa. Using histological and cytometric analyses, and paying careful attention to mycobacterial persistence and antigen-specific T cell priming, the inventors have defined the parameters required for achieving effective adaptive immune responses in the bladder. They have identified a requirement for live bacteria that disseminate to local draining lymph nodes in order to achieve T cell priming (Figure 3); and repeated instillations are needed to trigger recruitment of T cells to the bladder microenvironment (Figures 1-3). Careful analysis of these parameters has not been previously documented, in part due to inability to access patient material (e.g., bladder mucosa and lymph node) during a clinically approved therapeutic intervention.

Based on experimental work in humans, the inventors have focused their attention on the BCG induced inflammation and activation and recruitment of T lymphocytes after intravesical instillations in mice. Based on their observations of a delayed influx of T cells, they hypothesized that parenteral exposure to BCG prior to standard-of-care might accelerate the kinetics of bladder inflammation. They demonstrate that such an approach provides an optimized strategy for T cell recruitment and that this treatment protocol improves the host anti-tumor response

From the perspective of the host response to infection, one striking observation is that bladder T cell infiltration following repeated BCG instillations occurs only after day 29 (Figure 1C) showing similarity to what has been shown in the lung *Mtb* infection model. In their bladder instillation model, the number of BCG CFUs decays quickly (Figure 3A), however testing the response to higher dose of BCG remains technically challenging. It is worth noting that once established, the response is sustained, lasting at least 21 days following the third instillation.

The inventors also discovered that BCG dissemination to the regional lymph node is critical for achieving efficient T cell priming, again showing similarity to what has been shown in the lung *Mtb* infection model. T cell priming, however, was not sufficient to achieve T cell recruitment to the bladder, as shown by the relatively low level of T cell infiltration following a single instillation, even in the presence of measurable BCG-specific T cell responses (Figure 3F). To further assess the relationship between priming and trafficking to the bladder, they performed studies in mice that were previously primed *via* the subcutaneous route. These data demonstrated that trafficking of T cells to the bladder could be dissociated from the route of priming, in contrast to what has been reported in the context of homing to the gut or central nervous system. Bladder T cell recruitment correlated with a robust, but short-lived innate immune response, which is suggestive of a delayed type-hypersensitivity (DTH) response. The inventors report here that s.c. immunization 21 days prior to BCG intravesical instillation results in a more robust inflammatory response following intravesical BCG, which is dependent on T cells (Figure 5), thereby suggesting that bladder inflammation should be considered a DTH reaction. Although they demonstrate a critical role for primed T cells in the BCG-mediated influx of inflammatory innate cells, they were unable to define the cellular mechanism(s) governing T cell entry into the bladder. Notably, depletion of neutrophils, monocytes and NK cells cells did not result in impaired T cell trafficking to the bladder.

To apply their insights into dynamics of bladder inflammation, they tested their modified treatment regimen using an orthotopic bladder tumor model. Most strikingly, they demonstrate the ability to achieve up to 100% survival as compared to 80% lethality at day 70 (median survival time being ∼45 days) (Figure 7). These data are remarkable given the aggressive nature of MB49, but even more so for the ease of translating our results for testing in human clinical trials. Supporting the important role for pre-treatment BCG-specific responses, they conducted a retrospective study and identified absence of a PPD response to be a risk factor for treatment failure (Figure 8). In light of their data, they suggest that the first cycle of BCG might serve to prime patients, thus enhancing bladder inflammation and the chance to achieve tumor clearance during subsequent rounds of intravesical treatment.

In summary, the inventors have demonstrated that while BCG dissemination to regional LNs and priming of IFNγ-producing T cells can occur following a single instillation, repeated instillations of live BCG are necessary to achieve robust bladder T cell infiltration. Strikingly, parenteral exposure to BCG prior to instillation overcomes the requirement for repeated instillations, triggering a more robust acute inflammatory process at the first instillation and accelerating the recruitment of T cells to the bladder. Moreover, parenteral exposure to BCG prior to orthotopic tumor challenge dramatically improves response to BCG therapy. Importantly, patients with pre-existing immunity to BCG responded significantly better to therapy. Together these data suggest that checking patients' immunity to BCG prior to intravesical therapy, and boosting it if necessary, might improve BCG-induced clinical responses.

## Claims

1. A first and a second identical or different mycobacterial immunogenic compositions, each comprising at least a *Mycobacterium bovis* bacillus Calmette-Guérin (BCG), an antigenically related non-pathogenic mycobacteria, or one or more immunogenic component(s) thereof, as therapeutic active ingredient(s) for use in the treatment of cancer by parenteral or oral administration of the first composition to a cancer patient before local administration of the second composition at tumor site.

2. The first and/or second compositions according to claim 1, wherein said antigenically related non-pathogenic mycobacteria is selected from the group consisting of a recombinant BCG, a genetically modified *Mycobacterium tuberculosis* that has been attenuated through deletion of virulence genes, *Mycobacterium microti, Mycobacterium smegmatis, Mycobacterium fortuitum*, and *Mycobacterium vaccae.*

3. The first and/or second compositions according to claim 2, wherein the recombinant BCG is expressing one or more of a Th1 cytokine, a BCG or *Mycobacterium tuberculosis* immunodominant antigen, and listeriolysin.

4. The first and/or second compositions according to claim 1, wherein said BCG is selected from the group consisting of Pasteur, Frappier, Connaught, Tice, RIVM, and Danish 1331 strains.

5. The first and/or second compositions according to any one of claims 1 to 4, wherein the immunogenic component(s) of the non-pathogenic mycobacteria are selected from the group consisting of a mycobacterial cell wall fraction, a *Mycobacterium tuberculosis* or BCG recombinant immunodominant antigen, and a recombinant vector expressing said antigen(s).

6. The first and/or second compositions according to any one of claims 1 to 5, further comprising one or more additional agents chosen from pro-inflammatory agents, T-cell stimulatory molecules, antibiotics, and chemotherapy drugs.

7. The first composition according to claim 5 or claim 6, which is for the parenteral or oral administration to a patient having an active immune response to BCG.

8. The first and/or second compositions according to any one of claims 1 to 6, wherein the first composition comprises live or killed but metabolically active non-pathogenic mycobacteria.

9. The first composition according to claim 8, which is for the parenteral or oral administration to a patient having no active immune response to BCG.

10. The first and/or second composition according to any one of claims 1 to 9, wherein the second composition comprises live, killed but metabolically active, or killed and metabolically inactive non-pathogenic mycobacteria

11. The first and/or second compositions according to any one of claims 1 to 10, wherein the parenteral or oral administration is at least seven days before the local administration.

12. The first and/or second compositions according to any one of claims 1 to 11, wherein the parenteral or oral administration is oral, subcutaneous, percutaneous, or intradermal.

13. The first and/or second compositions according to any one of claims 1 to 12, wherein said local administration comprises at least three separate administrations at one to three weeks interval.

14. The first and/or second compositions according to any one of claims 1 to 13, wherein the cancer is a non-invasive mucosal cancer.

15. The composition according to claim 14, wherein said cancer is selected from the group consisting of carcinoma *in situ* and high-grade Ta or T1 transitional cell carcinoma of the bladder.

16. A method *in vitro* for monitoring cancer treatment by immunotherapy with BCG or antigenically related non-pathogenic mycobacteria, comprising:
- assaying BCG-specific immune response in a sample from a patient, wherein a positive assay correlates with an active anti-tumor response in the patient.

17. The method according to claim 16, wherein the BCG-specific immune response is assayed before to initiate the immunotherapy.

18. The method according to claim 16 or claim 17, wherein the BCG-specific immune response is assayed before tumor resection, before the first local administration, and/or at the end of the local administrations of the BCG, antigenically related non-pathogenic mycobacteria, or immunogenic component(s) thereof at tumor site.
